Europäisches Patentamt

European Patent Office

Office européen des brevets

(11) Publication number: **0 182 117**
B1

(12) EUROPEAN PATENT SPECIFICATION

(45) Date of publication of patent specification: **18.10.89**

(51) Int. Cl.⁴: **A 23 K 1/16**, A 23 K 1/17, A 23 K 1/18, A 61 K 31/52

(21) Application number: **85113315.7**

(22) Date of filing: **21.10.85**

(54) Anticoccidial compositions.

(30) Priority: **15.11.84 US 670621**

(43) Date of publication of application:
**28.05.86 Bulletin 86/22**

(45) Publication of the grant of the patent:
**18.10.89 Bulletin 89/42**

(84) Designated Contracting States:
**AT BE CH DE FR GB IT LI LU NL SE**

(56) References cited:
**EP-A-0 063 238**
**DE-A-2 154 049**
**GB-A-1 463 519**
**US-A-3 953 597**

(73) Proprietor: **AMERICAN CYANAMID COMPANY**
**1937 West Main Street P.O. Box 60**
**Stamford Connecticut 06904-0060 (US)**

(72) Inventor: **Kantor, Sidney**
**4A Martin Van Buren Drive**
**Cranbury, NJ 08512 (US)**

(74) Representative: **Wächtershäuser, Günter, Dr.**
**Tal 29**
**D-8000 München 2 (DE)**

Courier Press, Leamington Spa, England.

**Description**

## Background of the Invention

The present invention relates to compositions containing subtherapeutic doses of maduramicin and nicarbazin which synergistically control and/or prevent coccidiosis in poultry.

Both maduramicin and nicarbazin have been reported as effective anticoccidial agents. For instance, nicarbazin has been used as an anticoccidial but has been reported as inhibiting growth (German Patent 2,154,049). As such, attempts to alleviate the negative aspects of nicarbazin use such as growth inhibition or other safety concerns, have been and continue to be pursued. Therefore, combinations utilizing nicarbazin with other therapeutic compounds have been developed. For instance, combinations of nicarbazin with guanidines are disclosed in German Patent 2,154,049; as well as purines formulated with monensin and nicarbazin being disclosed in U.K. Patent 1,463,519; further, metichorpindol in combination with 4-hydroxyquinolines is disclosed in U.S. Patent 3,761,594.

Maduramicin, on the other hand, is a new antibiotic that has been shown to be an effective anticoccidial agent (U.S. Patent 4,278,663; U.S. Patent 4,368,265; and U.S. Patent 4,407,946). Although combinations of nicarbazin with polyether antibiotics are disclosed in U.S. Patent 4,218,438 and Reexamined U.S. Patent B4,218,438, compositions containing maduramicin and nicarbazin at extremely low subtherapeutic doses for effective control of coccidiosis in poultry have not been disclosed.

## Summary of the Invention

The present invention unexpectedly has been found to synergistically control and/or prevent coccidiosis in poultry by combining the relatively new anticoccidial agent, maduramicin, with nicarbazin. Not only that, but the present invention also relates to a method for preventing and/or controlling said disease by orally administrating maduramicin and nicarbazin to poultry in compositions which synergistically control said coccidiosis.

It is an object of this invention, therefore, to provide a method whereby coccidiosis is controlled and/or prevented in poultry by utilizing compositions containing maduramicin and nicarbazin. It is a further object of the present invention to provide such compositions wherein said drugs are present in previously considered subtherapeutic doses and wherein said drugs synergistically control and/or prevent coccidiosis.

These and further objects will become more apparent by the following more detailed description of the invention.

## Description of the Invention

The compositions useful in the present invention contain both components at subtherapeutic doses. By subtherapeutic is meant a dose which is less than that normally required in therapeutic effect when the component is used alone or as usually combined with a polyether antibiotic or nicarbazin. The dose according to the invention is, of course, therapeutic. For instance, maduramicin is usually orally administered to poultry at doses of about 5 ppm or more. However, in the present feedstuff compositions, doses as low as 1.00 ppm are useful in controlling coccidiosis in poultry. Thus, maduramicin is present at levels of 1.00 ppm to 8.00 ppm, preferably 1.25 ppm to 5.00 ppm, and most preferred 2.50 ppm to 5.00 ppm.

Likewise, nicarbazin's usual dose level, when orally administered in feedstuff compositions to poultry, is 125 ppm. Animal feedstuff compositions of the present invention contain nicarbazin at levels of 1.5 to 125 ppm, preferably 1.5 to 50 ppm, and most preferred 1.5 ppm to 25 ppm. Therefore, combination dosages of therapeutic agents present in animal feedstuff compositions of the present invention contain as little as 2.50 ppm to 130 ppm, preferably 3.75 ppm to 55 ppm, most preferred 4.00 ppm to 30 ppm drugs, whereas other compositions containing nicarbazin with polyether antibiotics contain 40 ppm to 245 ppm of total drugs. Thus, the combination dose of the present invention is subtherapeutic to those of other compositions.

These levels of maduramicin and nicarbazin demonstrate significant control of coccidiosis caused by strains of *Eimeria,* as evidenced by increased survival of chickens and decreased lesion scores. Further, no adverse effects on weight gain are observed in the treated birds.

Animal feedstuff compositions containing the therapeutic and/or prophylactic levels of maduramicin and nicarbazin may be readily prepared by admixing these drugs with the feedstuff directly or by admixing a premix containing one or both of the drugs with the desired feedstuff.

Poultry feedstuffs of all types and formulae normally used in the poultry industry are appropriate for use in the present invention. The following provide examples of such formulae.

Broiler Starter

| Ingredients | Weight Percent |
|---|---|
| Corn, Yellow, Ground | 50.0 |
| Soybean Oil Meal, Solvent Extracted Dehulled (50%) | 30.9 |
| Animal Fat | 6.5 |
| Fish Meal with Solubles (60%) | 5.0 |
| Corn Distillers Dried Solubles | 4.0 |
| Dicalcium Phosphate, Feed Grade | 1.8 |
| Calcium Carbonate (Ground Limestone) | 0.8 |
| Vitamin Premix TK-01 (1.03) | 0.5 |
| Salt (NaCl) | 0.3 |
| Trace Mineral Premix TK-01 (1.02) | 0.1 |
| Methionine Hydroxy Analog | 0.1 |
| Total | 100.0 |

Broiler Grower

| Ingredients | Weight Percent |
|---|---|
| Corn, Yellow, Ground | 57.7 |
| Soybean Meal, Solvent, Extracted dehulled (50%) | 31.7 |
| Animal Fat (Beef tallow) | 6.0 |
| Dicalcium Phosphate, Feed Grade | 2.7 |
| Calcium Carbonate (Ground limestone) | 0.9 |
| Vitamin Premix TK-01 (1.03) | 0.5 |
| Salt (NaCl) | 0.2 |
| Methionine Hydroxy Analog | 0.2 |
| Trace Mineral Premix TK-01 (1.02) | 0.1 |
| Total | 100.0 |

# EP 0 182 117 B1

## Chick Starter, Light Breeds

| Ingredients | Weight Percent |
|---|---|
| Corn, Yellow, Ground | 56.3 |
| Soybean Meal, Solvent Extracted, Dehulled (50%) | 17.9 |
| Wheat Middlings | 10.0 |
| Corn Distillers Dried Solubles | 5.0 |
| Fish Meal with Solubles | 5.0 |
| Alfalfa Meal, Dehydrated (17%) | 2.5 |
| Dicalcium Phosphate, Feed Grade | 1.3 |
| Calcium Carbonate | 0.9 |
| Vitamin Premix | 0.5 |
| Salt (NaCl) | 0.3 |
| Methionine Hydroxy Analog | 0.2 |
| Trace Mineral Premix | 0.1 |
| Total | 100.0 |

## Pullet Grower

| Ingredients | Weight Percent |
|---|---|
| Corn, Yellow, Ground | 73.5 |
| Soybean Meal, Solvent Extracted, Dehulled (50%) | 21.9 |
| Dicalcium Phosphate, Feed Grade | 2.5 |
| Calcium Carbonate | 1.0 |
| Vitamin Premix | 0.5 |
| Salt (NaCl) | 0.3 |
| Methionine Hydroxy Analog | 0.2 |
| Trace Mineral Premix | 0.1 |
| Total | 100.0 |

# EP 0 182 117 B1

### Pullet Developer

| Ingredients | Weight Percent |
|---|---|
| Corn, Yellow, Ground | 67.5 |
| Oats, Ground Whole | 15.0 |
| Soybean Meal, Solvent Extracted, Dehulled (50%) | 13.4 |
| Dicalcium Phosphate, Feed Grade | 2.1 |
| Calcium Carbonate | 1.0 |
| Vitamin Premix | 0.5 |
| Methionine Hydroxy Analog | 0.3 |
| Salt (NaCl) | 0.2 |
| Trace Mineral Premix | 0.1 |
| Total | 100.1 |

### Turkey Starter

| Ingredients | Weight Percent |
|---|---|
| Soybean Meal, Solvent Extracted, Dehulled | 40.7 |
| Corn, Yellow, Ground | 39.7 |
| Fish Meal with Solubles | 5.0 |
| Beef Tallow | 5.0 |
| Corn Distillers Dried Solubles | 2.5 |
| Alfalfa Meal, Dehydrated (17%) | 2.5 |
| Dicalcium Phosphate, Feed Grade | 2.5 |
| Calcium Carbonate | 1.2 |
| Vitamin Premix | 0.5 |
| Salt (NaCl) | 0.2 |
| Trace Mineral Premix | 0.1 |
| Methionine Hydroxy Analog | 0.1 |
| Total | 100.0 |

Turkey Finisher

| Ingredients | Weight Percent |
|---|---|
| Corn, Yellow, Ground | 71.2 |
| Soybean Meal, Solvent Extracted Dehulled (50%) | 9.9 |
| Corn Distillers Dried Solubles | 5.0 |
| Alfalfa Meal, Dehydrated (17%) | 5.0 |
| Animal Fat | 3.0 |
| Fish Meal with Solubles | 2.5 |
| Dicalcium Phosphate, Feed Grade | 1.7 |
| Calcium Carbonate | 0.5 |
| Vitamin Premix | 0.5 |
| Salt (NaCl) | 0.4 |
| Methionine Hydroxy Analog | 0.2 |
| Trace Mineral Premix | 0.1 |
| Total | 100.0 |

The anticoccidial activity of compositions of the present invention are illustrated by the following nonlimiting examples.

Example 1

Preparation of a premix composition of maduramicin

The ammonium salt of maduramicin (55 g, 1.1% on a weight basis) is dissolved in 385 g, 7.7% on a weight basis of benzyl alcohol, thereby forming a murky suspension. This suspension is fed in a fine stream into a ribbon blender (one cubic foot capacity) holding 4,460 g, 89.2% on a weight basis of granular corncob (Grit-O-Cob) and is followed by the addition of 100 g, 2.0% on a weight basis, oil as a rinse of the container. The mixture is allowed to blend until uniform, about ten minutes, and a free-flowing dry granular product results.

Example 2

Preparation of medicated poultry feed containing nicarbazin and maduramicin

Sufficient quantities of a 1% by weight maduramicin premix and 25% commercially available nicarbazin premix containing wheat middlings and soybean oil are admixed until homogeneous with a poultry diet containing the ingredients listed in Table I below, to provide medicated feed compositions containing from 1.5 ppm to 125 ppm of nicarbazin and 1.25 ppm to 5 ppm of maduramicin. In these examples, when 5.0 ppm of maduramicin is preferred, 5% of a 1% maduramicin premix is mixed with nicarbazin in a feedstuff. Of course, this quantity changes according to the amount of drug used for testing. Likewise, when 125 ppm of nicarbazin are required, 0.05% of a 25% premix is utilized. Again, this percentage changes with the amount of nicarbazin used in testing.

## Table I

| Ingredients | Weight Percent |
|---|---|
| Vitamin-amino acid premix | 0.5 |
| Trace minerals | 0.1 |
| Sodium chloride | 0.3 |
| Dicalcium phosphate | 1.2 |
| Ground limestone | 0.5 |
| Stabilized fat | 4.0 |
| Dehydrated alfalfa, 17% protein | 2.0 |
| Corn gluten meal, 41% protein | 5.0 |
| Menhaden fish meal, 60% protein | 5.0 |
| Soybean oil meal, 44% protein | 30.0 |
| Ground yellow corn, fine to | 100.0 |

The vitamin-amino acid premix in the above feed composition is prepared from the following formulation. The expressions of quantity relate to units per kilogram of the finished feed composition.

| | | |
|---|---|---|
| Butylated hydroxytoluene | 125.0 | mg |
| dl-Methionine | 500.0 | mg |
| Vitamin A | 3300.0 | I.U. |
| Vitamin $D_3$ | 100.0 | I.C.U. |
| Riboflavin | 4.4 | mg |
| Vitamin E | 2.2 | I.U. |
| Niacin | 27.5 | mg |
| Pantothenic acid | 8.8 | mg |
| Choline chloride | 500.0 | mg |
| Folic acid | 1.43 | mg |
| Menadione sodium bisulfate | 1.1 | mg |
| Vitamin $B_{12}$ | 11.0 | mcg |
| Ground yellow corn to | 5.0 | g |

For the examples used in the test compositions of the present invention, the following provide the quantity amounts of maduramicin and/or nicarbazin in mg per kg of feed.

7

| ppm | (mg/kg) 25% Nicarbazin Premix | (mg/kg) 1% Maduramicin Premix |
|---|---|---|
| 1.25 | — | 125 |
| 1.50 | 6 | — |
| 2.50 | — | 250 |
| 5.00 | — | 500 |
| 6.00 | 24 | — |
| 12.50 | 50 | — |
| 25.00 | 100 | — |
| 50.00 | 200 | — |
| 85.00 | 340 | — |
| 125.00 | 500 | — |

Example 3

Evaluation of test compositions as anticoccidial agents

Anticoccidial activity of maduramicin and nicarbazin compositions administered to chickens is demonstrated in the test described herein.

The poultry diets prepared as in Example 2 are the ones utilized in this experiment.

A sufficient number of oocysts of *Eimeria tenella* to produce 60% to 75% mortality in untreated controls were given to one-day-old chicks, by direct inoculation into the crops of all chicks. The chicks were given free access to feed and water during the entire test period. Two days before inoculation, medicated feed, with several levels of drug, was presented to the various groups of chicks. The chicks were weighed 3 days after inoculation. Then seven days after inoculation, the experiments were terminated, and the chicks were weighed, sacrificed and their intestinal tracts were examined for lesions. The results appear in Table II.

TABLE II

A. % Survivability

Eimeria strain

| Nicarbazin ppm | Maduramicin ppm | | | | |
|---|---|---|---|---|---|
| | 0.0 | 1.25 | 2.5 | 3.75 | 5.0 |
| 0.0 | 47.0 | 67.0 | 100.00 | 100.00 | 100.00 |
| 1.5 | -- | -- | -- | 100.00 | 100.00 |
| 3.0 | -- | -- | -- | 100.00 | 100.00 |
| 6.0 | -- | -- | -- | 100.00 | 100.00 |
| 12.5 | 53.0 | -- | -- | 100.00 | 100.00 |
| 25.0 | 67.0 | -- | -- | 100.00 | 100.00 |
| 50.0 | 80.0 | 100.00 | 100.00 | -- | 100.00 |
| 85.0 | 93.0 | 100.00 | -- | -- | -- |
| 125.0 | 100.00 | -- | -- | -- | 100.00 |

B. % Weight gain - three to seven days

Eimeria strain

| Nicarbazin ppm | Maduramicin ppm | | | | |
|---|---|---|---|---|---|
| | 0.0 | 1.25 | 2.5 | 3.75 | 5.0 |
| 0.0 | 38.0 | 67.0 | 78.00 | 104.00 | 98.00 |
| 1.5 | -- | -- | -- | 106.00 | 107.00 |
| 3.0 | -- | -- | -- | 110.00 | 110.00 |
| 6.0 | -- | -- | -- | 111.00 | 106.00 |
| 12.5 | 12.0 | -- | -- | 114.00 | 93.00 |
| 25.0 | 43.0 | -- | -- | 106.00 | 105.00 |
| 50.0 | 32.0 | 97.00 | 100.00 | -- | 109.00 |
| 85.0 | 93.0 | 72.00 | -- | -- | -- |
| 125.0 | 104.00 | -- | -- | -- | 90.00 |

# EP 0 182 117 B1

## TABLE II (Continued)

### C. Lesion scores

*Eimeria* strain

| Nicarbazin ppm | Maduramicin ppm | | | | |
|---|---|---|---|---|---|
| | 0.0 | 1.25 | 2.5 | 3.75 | 5.0 |
| 0.0 | 3.6 | 3.4 | 3.2 | 2.2 | 0.7 |
| 1.5 | -- | -- | -- | 0.3 | -- |
| 3.0 | -- | -- | -- | 0.1 | 0.4 |
| 6.0 | -- | -- | -- | 0.2 | 0.0 |
| 12.5 | 3.5 | -- | -- | 0.8 | 0.0 |
| 25.0 | 3.3 | -- | -- | 0.0 | 0.0 |
| 50.0 | 3.2 | 0.7 | 0.0 | -- | 0.0 |
| 85.0 | 1.1 | 0.0 | -- | -- | 0.0 |
| 125.0 | 0.5 | -- | -- | -- | 0.0 |

These results indicate not only improved survivability of infected chicks when treated with the present compositions and no detrimental effect on percent weight gain but also show significant suppression of lesions due to *E. tenella* infection with administration of the present compositions even at extremely subtherapeutic doses of drugs.

**Claims for the Contracting States: BE CH DE FR GB IT LI LU NL SE**

1. A poultry feedstuff composition characterized by: maduramicin and nicarbazin; wherein said maduramicin and nicarbazin are present at doses synergistically effective for controlling coccidiosis in poultry.

2. A composition according to Claim 1 characterized by concentrations of maduramicin of 2.50 ppm to less than 5.00 ppm and of nicarbazin of 1.50 ppm to 25 ppm.

3. A method for controlling and preventing coccidiosis in poultry, said method characterized by: orally administering to poultry a coccidiosis-controlling-and-preventing-amount of a composition containing maduramicin and nicarbazin, wherein said maduramicin and nicarbazin are synergistically effective in controlling and preventing coccidiosis in poultry and are present in said composition in combined subtherapeutic doses; wherein said subtherapeutic doses of maduramicin are in amounts of 1.00 ppm to less than 5.00 ppm and said subtherapeutic doses of nicarbazin are in amounts of 1.50 ppm to less than 125 ppm.

4. A method according to Claim 3, characterized by concentrations of maduramicin of 2.50 ppm to less than 5.00 ppm and of nicarbazin of 1.50 ppm to 25 ppm.

**Claims for the Contracting State: AT**

1. Use of maduramicin and nicarbazin in the preparation of a poultry feedstuff composition wherein maduramicin and nicarbazin are present at doses synergistically effective for controlling coccidiosis in poultry.

2. A use according to Claim 1, characterized by concentrations of maduramicin of 2.50 ppm to less than 5.00 ppm and of nicarbazin of 1.50 ppm to 25 ppm.

**Patentansprüche für die Vertragsstaaten: BE CH DE FR GB IT LI LU NL SE**

1. Geflügelfutterzusammensetzung, gekennzeichnet durch: Maduramicin und Nicarbazin; wobei die erwähnten Maduramicin und Nicarbazin in Dosismengen vorliegen, die synergistisch wirksam sind im Sinne einer Bekämpfung von Coccidiosis bei Geflügel.

2. Zusammensetzung gemäß Anspruch 1, gekennzeichnet durch Konzentrationen an Maduramicin von 2,50 TpM bis weniger als 5,00 TpM und von Nicarbazin von 1,50 TpM bis 25 TpM.

3. Verfahren zur Bekämpfung und Verhinderung von Coccidiosis bei Geflügel, wobei das Verfahren dadurch gekennzeichnet ist, daß man dem Geflügel eine Coccidiosis bekämpfende und verhindernde Menge einer Zusammensetzung verabreicht, welche Maduramicin und Nicarbazin enthält, wobei die erwähnten Maduramicin und Nicarbazin synergistisch wirksam sind bei der Bekämpfung und Verhinderung von Coccidiose bei Geflügel und in der Zusammensetzung in kombinierten subtherapeutischen Dosismengen vorliegen; wobei die erwähnten subtherapeutischen Dosismengen an Maduramicin Mengen von 1,00 TpM bis weniger als 5,00 TpM sind und die erwähnten subtherapeutischen Dosismengen an Nicarbacin Mengen von 1,50 TpM bis weniger als 125 TpM sind.

4. Verfahren gemäß Anspruch 3, gekennzeichnet durch Konzentrationen an Maduramicin von 2,50 TpM bis weniger als 5,00 TpM und an Nicarbazin von 1,50 TpM bis 25 TpM.

**Patentansprüche für den Vertragsstaat: AT**

1. Verwendung von Maduramicin und Nicarbazin bei der Herstellung einer Geflügelfutterzusammensetzung, wobei Maduramicin und Nicarbacin in Dosismengen vorliegen, die synergistisch wirksam sind im Sinne einer Bekämpfung von Coccidiosis bei Geflügel.

2. Verwendung gemäß Anspruch 1, gekennzeichnet durch Konzentrationen an Maduramicin von 2,50 TpM bis weniger als 5,00 TpM und an Nicarbazin von 1,50 TpM bis 25 TpM.

**Revendications pour les Etats contractants: BE CH DE FR GB IT LI LU NL SE**

1. Composition alimentaire pour la volaille, caractérisée en ce qu'elle contient de la maduramicine et de la nicarbazine, la maduramicine et la nicarbazine étant présentes selon des doses efficaces pour lutter, de manière synergique, contre la coccidiose chez la volaille.

2. Composition selon la revendication 1, caractérisée par des concentrations en maduramicine de 2,50 ppm jusqu'à moins de 5,00 ppm, et en nicarbazine, de 1,50 ppm à 25 ppm.

3. Procédé pour lutter contre, et prévenir la coccidiose chez la volaille, caractérisé en ce qu'on administre par voie orale à la volaille, une quantité efficace pour lutter contre et prévenir la coccidiose, d'une composition contenant de la maduramicine et de la nicarbazine, la maduramicine et la nicarbazine étant efficaces, en synergie, pour lutter contre et prévenir la coccidiose chez la volaille, et étant présente selon des doses combinées subthérapeutiques dans cette composition; ces doses subthérapeutiques maduramicine étant de 1,00 ppm à moins de 5,00 ppm, et ces doses subthérapeutiques de nicarbazine étant de 1,50 ppm à moins de 125 ppm.

4. Procédé selon la revendication 3, caractérisé par des concentrations en maduramicine de 2,50 ppm à moins de 5,00 ppm, et en nicarbazine de 1,50 ppm à 25 ppm.

**Revendications pour l'Etat contractant: AT**

1. Utilisation de maduramicine et de nicarbazine pour la préparation d'une composition alimentaire pour la volaille, contenant de la maduramicine et de la nicarbazine selon des doses efficaces pour lutter, de manière synergique, contre la coccidiose de la volaille.

2. Utilisation selon la revendication 1, caractérisée par des concentrations en maduramicine de 2,50 ppm à moins de 5,00 ppm, et en nicarbazine, de 1,50 ppm à 25 ppm.